# EUROPEAN PATENT SPECIFICATION

(11) **EP 3 641 863 B1**
(45) Date of publication and mention of the grant of the patent: **20.01.2021**
(21) Application number: 18731103.0
(22) Date of filing: 19.06.2018
(51) Int. Cl.: A61M 15/00, A61M 11/00

(54) **DOSING SYSTEM FOR AN INHALATION DEVICE, INHALATION DEVICE, AND METHOD FOR DOSING LIQUID TO AN INHALATION DEVICE**
DOSIERSYSTEM FÜR EINE INHALATIONSVORRICHTUNG, INHALATIONSVORRICHTUNG, UND VERFAHREN ZUR DOSIERUNG VON FLÜSSIGKEIT ZU EINER INHALATIONSVORRICHTUNG
SYSTÈME DE DOSAGE POUR UN DISPOSITIF D'INHALATION, DISPOSITIF D'INHALATION, ET PROCÉDÉ DE DOSAGE D'UN LIQUIDE À UN DISPOSITIF D'INHALATION

(30) Priority: 21.06.2017 EP 17177225
(43) Date of publication of application: 29.04.2020
(73) Proprietor: Vectura Delivery Devices Limited, Chippenham, Wiltshire SN14 6FH (GB)
(72) Inventor: GOODWIN, Paul, Cambridge Cambridgeshire CB4 0GZ (GB); MELINIOTIS, Andreas Mark, Cambridge Cambridgeshire CB4 0GZ (GB); CLARKE, Roger William, Cambridge Cambridgeshire CB4 0GZ (GB); KOLB, Tobias, Cambridge Cambridgeshire CB4 0GZ (GB)
(74) Representative: Clarke, Christopher John
(86) International application number: PCT/EP2018/066296
(87) International publication number: WO 2018/234324

(56) References cited:
- EP-A1- 1 205 199
- EP-A2- 0 388 106
- WO-A1-03/061741
- WO-A1-2011/055243
- WO-A1-2015/022436
- CN-U- 204 995 925
- JP-B2- 3 563 467
- US-A1- 2007 068 593

## Description

### Introduction

Nebulizers are inhalation devices that convert a liquid formulation, which usually contains an active agent, into an inhalable aerosol (i.e. a dispersion of fine liquid droplets), for example by means of an ultrasonic aerosol generator, a jet or a vibrating mesh. The aerosol is delivered to the lungs by inhalation, particularly for the treatment of respiratory diseases such as asthma and cystic fibrosis. Nebulizers differ from other inhalation devices such as dry powder inhalers, pressurized metered dose inhalers and soft mist inhalers in that they operate continuously. Treatment may take place during a few breaths or for an extended period of time (e.g. up to about 45 minutes). During this time, the nebulizer emits aerosol either constantly or in pulses which may be adapted to the user's breathing pattern; for example, aerosol generation may be triggered by the onset of inhalation. Thus, nebulizers do not *per se* emit metered amounts of aerosols, and unless switched off, they produce aerosol until the liquid has all been used up.

Consequently, it is necessary to dose the correct amount of liquid formulation to the aerosol generator. One way of doing this is to use pre-filled single-use cartridges which are completely emptied into the nebulizer, so that the liquid is all nebulized. However, the dosing flexibility of such cartridges is limited because a particular cartridge can only dose one fixed volume. Thus when the prescribed amount of medicine to be inhaled does not match the volume of the liquid supplied in the container, it is necessary to ensure that only the prescribed amount is delivered in aerosol form.

### Background to the Invention

A dosing system for this purpose is disclosed in EP 1465 692, having a metering chamber and a second (overflow) chamber. The metering chamber defines the volume of the substance to be nebulized and is arranged so as to feed this volume to the aerosol generator, while any substance poured into the metering chamber in excess of its volume is received and retained in the second chamber. In other words, the metering chamber is filled until the liquid overflows into the second chamber, and only the metered volume inside the metering chamber is subsequently nebulized. This has the disadvantage that any changes in the prescribed dose would require complete replacement of the metering chamber assembly. Furthermore, the metering system is not suitable for metering very small amounts of liquids which are substantially affected by adhesive and cohesive forces and do not easily flow from one chamber to another.

Further dosing systems are disclosed in EP 1 205 199 and EP 2 496 293. Both of these have a filling chamber with a wider upper portion and a narrower lower portion that is closed by a valve at its bottom end. A plunger is inserted into the filling chamber from its wider upper end along the chamber's longitudinal axis. Once the plunger reaches the narrower lower portion, a seal is formed between the plunger and the walls of the lower portion, so that liquid can no longer be displaced upwards into the upper portion. Upon continued insertion of the plunger, the liquid in the lower portion is pushed out through the valve, thus dosing a metered volume, while the excess liquid remains in the upper portion above the seal. The dispensed volume can be altered by changing the volume and / or the extent of insertion of the plunger. The plunger actively displaces the liquid to be dosed, thus overcoming the issues associated with dispensing small amounts of viscous liquids. When the plunger is retracted, the excess liquid can flow into the lower portion and could be pushed out through the valve if the plunger is re-inserted. This is advantageous when the filling chamber is deliberately filled with a multi-dose amount of liquid and the dosing system is supposed to be actuated repeatedly. However, it is highly undesirable in cases where such re-dosing is unintended and may even be harmful due to overdosing. For example, only small amounts (substantially less than the supplied volume) of liquid formulation may be intended to be administered to neonates, infants, or children, or to subjects with an improving health-condition. For instance, the liquid formulation may only be available in ampoules containing 1 mL or more, while the subject should receive only 200 µL. The dosing systems of EP 1 205 199 and EP 2 496 293 would allow the unintended administration of an extra 800 µL to the patient.

WO 2015/022436 discloses a dosing system having both an overflow chamber and a plunger which forms a seal with the filling chamber, in order to isolate the excess volume of liquid that is not supposed to be administered to the user, so that it cannot be re-dosed accidentally. Two general types of dosing system are disclosed. In the first, the filling chamber is separated from the aerosol generator chamber by a closing means, such as a duckbill valve. Liquid is poured into the filling chamber, where it is retained by the valve. The plunger is inserted, thereby displacing some of the liquid into the overflow chamber. The plunger must then form a seal with the filling chamber wall, so that it can apply pressure to the liquid in order to open the valve and supply a metered volume of liquid to the aerosol generator chamber. In the second type of dosing system, there is no valve between the filling chamber and aerosol generator; nonetheless a seal between the plunger and the filling chamber is necessary, in order to isolate a metered volume of liquid. However, the requirement of forming a seal means imposes requirements on the materials from which the plunger and / or filling chamber are made, and / or require additional components, such as O rings.

When the user opens the lid of the dosing system (as in EP 1 465 692) and / or removes the plunger (as in EP 1 205 199, EP 2 496 293 and most of the embodiments of WO 2015/022436) after nebulization, the excess liquid is visible to the user. As a result, the user may mistakenly think that they have not received the full dose, and thus may try to use the excess, which could result in an overdose. Alternatively, the user might understand that they have received the correct dose, but then try to use the excess liquid for a subsequent dose, in order not to waste the liquid, which could result in the incorrect dose and also lead to contamination.

Thus there is a need for an improved dosing system which can accurately dispense pre-determined volumes of liquid, especially small amounts, which does not suffer from the drawbacks of the previous dosing systems.

### Brief Description of the Invention

In a first aspect, the present invention provides a dosing system for an inhalation device, comprising:
(a) a filling chamber for receiving a liquid to be aerosolized, the filling chamber having an outer wall, a base and an inner wall which defines an outlet opening,
(b) a reservoir chamber for supplying liquid an aerosol generator,
(c) a plunger which is mounted on a hinge and which includes an overflow chamber,
wherein the inner wall of the filling chamber is higher on the side adjacent to the hinge than on the opposite side so that when the filling chamber is filled with liquid and the plunger is inserted into the filling chamber by pivoting it about the hinge, part of the liquid is displaced by the plunger over the lower side of the inner wall of the filling chamber and into the reservoir chamber via the outlet opening, and some or all of the remaining liquid is displaced by the plunger from the filling chamber into the overflow chamber.

Preferably the dosing system comprises a cap located above the filling chamber outlet opening which prevents liquid from being supplied directly into the reservoir chamber.

Preferably the plunger has an inner wall and an outer wall, and at least part of the inner and outer walls of the filling chamber and the plunger are curved in profile so that there is a close fit between the inner walls of the plunger and filling chamber when the plunger is fully inserted into the filling chamber.

Preferably the hinge is provided with a detent mechanism which resists the pivoting motion of the plunger in order to prevent the plunger from being inserted rapidly, which could cause some of the liquid to splash out of the filling chamber.

Preferably the top of the lower side of the inner wall of the filling chamber and the top of the inner wall of the plunger are at the same height when the plunger is fully inserted.

Preferably a partition is located inside the inner wall of the filling chamber, which more preferably is parallel to the hinge and even more preferably extends vertically down into the reservoir chamber. The liquid which is displaced from the filling chamber when the plunger is inserted flows over the lower part of the inner wall and down the corresponding side of the reservoir chamber. The higher part of the inner wall prevents liquid from flowing down on the opposite side, so that air is displaced upwardly on that side of the reservoir chamber. This prevents the formation of an airlock, i.e. a trapped bubble of air at the bottom of the reservoir chamber.

Conveniently, the cap can be formed as an extension of the partition.

Preferably the overflow chamber has a cover on the side adjacent to the hinge, which closes the top of the overflow chamber on this side. More preferably the cover comprises a semi-annular wall and a semi-annular floor which preferably slopes slightly downwards from the outer wall of the overflow chamber adjacent to the hinge towards the opposite side of the overflow chamber (when the plunger is inserted). Thus, as the plunger is inserted, excess liquid is displaced over the lower side of the filling chamber inner wall and enters the uncovered part of the overflow chamber. The higher part of the filling chamber inner wall, together with the cover wall prevents liquid from flowing onto the top of the cover floor, or at least minimizes the amount of liquid that flows on to the top of the cover floor. Nonetheless, the slope of the cover floor guides any liquid which passes over or round the higher part of the filling chamber inner wall, or over the filling chamber outer wall, back down the slope towards the uncovered part of the over flow chamber opening and into the overflow chamber. Moreover, when the plunger is opened after nebulization has been completed, the cover floor is in a generally vertical orientation. In this position, the cover prevents liquid from flowing out of the overflow chamber. The cover closes enough of the top of the overflow chamber so that the liquid cannot flow out when the plunger is pivoted into the open position.

Preferably the plunger has a lid which covers the top of, and prevents direct access to, the overflow chamber, so that liquid cannot be filled directly into the overflow chamber, and so that the excess liquid is not visible to the user after nebulization. The lid is preferably fixed so that it is difficult for the user to access the overflow chamber. In order to allow the overflow chamber to be emptied and cleaned after nebulization has been completed, there is preferably an opening between the lid and the top of the overflow chamber. Preferably the opening is formed without a spout or other means of facilitating pouring out of the liquid, so that it is possible, but somewhat awkward, for the user to empty the overflow chamber. This emphasizes to the user that the excess liquid is not intended to be re-used.

Alternatively, the lid may be removable or openable, which facilitates emptying and cleaning of the overflow chamber after use. For example, the overflow chamber may be separately pivotable. Preferably the overflow chamber and lid have clip formations so that when the lid is closed, it becomes attached to the overflow chamber.

Preferably the overflow chamber corresponds to the size and shape of the filling chamber, so that the plunger forms a close fit and preferably occupies substantially the whole of the filling chamber when inserted. Nonetheless, when the filling chamber has an inner wall, there is preferably a small gap between the inner wall of the filling chamber and plunger (when inserted), through which the liquid displaced from the filling chamber flows. Preferably the gap is from 0.1 to 0.2mm in width, such as about 0.15mm.

Preferably the filling chamber and overflow chamber are generally annular in shape. Preferably the plunger is shaped so that it cannot be inserted into the reservoir chamber.

Preferably the filling chamber and the plunger are made from a rigid material, such as a rigid plastic.

In a second aspect, the present invention provides an inhalation device comprising the dosing system of the first aspect of the invention.

Preferably the inhalation device comprises an aerosol head and a base unit which are detachably connectible with each other, and wherein the aerosol head comprises the dosing system. More preferably, the aerosol head and base unit have complementary male and female features which interlock to provide a recognition system, for example, the base unit has two pegs of different sizes and the aerosol head has two corresponding holes.

In a third aspect, the present invention provides a method for dosing liquid to an inhalation device according to the second aspect of the invention, the method comprising supplying a liquid to be aerosolized to the filling chamber; and inserting the plunger into the filling chamber so that part of the liquid is displaced over the lower side of the inner wall of the filling chamber and into the reservoir chamber, and some or all of the remaining liquid is displaced into the overflow chamber.

### Detailed Description of the Invention

The present invention is further described with reference to the drawings, in which:
**Figures 1, 2****,** **3** and **4** illustrate the general principle of a dosing system
**Figure 5** shows side views of a dosing system according to the invention before and after the plunger is inserted into the filling chamber
**Figure 6** shows cross-sectional views which correspond to the side views of Figure 5
**Figure 7** shows a front view of the dosing system of Figure 5 before the plunger is inserted into the filling chamber
**Figure 8** shows a corresponding view of the dosing system of Figure 5 from above
**Figure 9** is an expanded view showing the components of the dosing system of Figure 5
**Figure 10** shows the operation of the dosing system of Figure 5
**Figure 11** shows a nebulizer device
**Figure 12** shows the aerosol generator of the nebulizer device of Figure 11
**Figure 13** shows a recognition system for the nebulizer device of Figure 11

### List of numerical references used in the Figures

| | | | |
|---|---|---|---|
| 1 | Dosing system | 57 | Curved region of plunger outer wall |
| 3 | Liquid | 61 | Cover |
| 10 | Filling chamber | 62 | Cover floor |
| 11 | Filling chamber outer wall | 63 | Cover wall |
| 12 | Filling chamber inner wall | 64 | Uncovered region of overflow chamber |
| 13 | Filling chamber base | 100 | Base unit |
| 14 | Filling chamber outlet opening | 102 | Air outlet opening |
| 15 | Central space | 103 | Groove |
| 16 | Higher side of inner wall | 104 | Base unit key lock members |
| 17 | Lower side of inner wall | 106 | Indentation |
| 20 | Plunger | 140 | Pegs |
| 21 | Plunger outer wall | 200 | Mouthpiece |
| 22 | Plunger inner wall | 201 | Air inlet opening |
| 23 | Plunger base | 202 | Lateral opening |
| 25 | Overflow chamber | 203 | Aerosol outlet opening |
| 26 | Gap | 204 | Positioning member |
| 27 | Lid | 300 | Aerosol head |
| 28 | Overflow chamber outlet opening | 301 | Aerosol generator |
| 31 | Reservoir chamber | 303 | Aerosol head key lock members |
| 34 | Perforated membrane | 306 | Transducer body |
| 35 | Partition | 308 | Piezoelectric member |
| 37 | Cap | 310 | Filling chamber |
| 50 | Hinge | 328 | Screw thread |
| 51 | Curved region of filling chamber inner wall | 331 | Reservoir |
| 52 | Curved region of plunger inner wall | 334 | Perforated membrane |
| 53 | Straight region of filling chamber inner wall | 340 | Holes |
| 54 | Straight region of plunger inner wall | | |
| 55 | Cut-away region | | |
| 56 | Curved region of filling chamber outer wall | | |

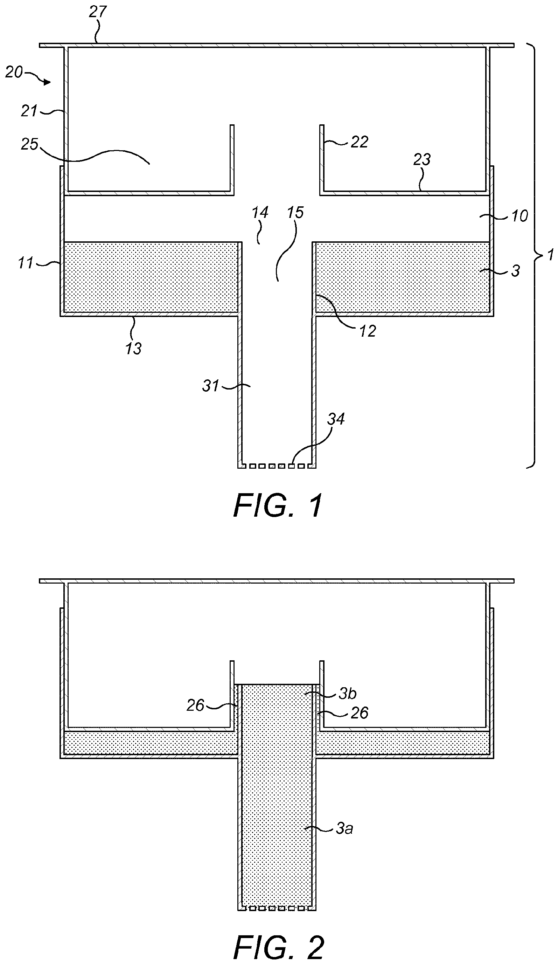

Figures 1 to 4 (not according to the invention) illustrate the general principle of a dosing system. The dosing system **1** has a filling chamber **10** for receiving the liquid **3** to be nebulized. The filling chamber has an outer wall **11,** an inner wall **12** and a base **13,** and is open at its upper end.

The outer and inner walls are circular (when viewed from above), so that the filling chamber **10** is annular. The top of the inner wall **12** forms an outlet opening **14.** The inner wall **12** also defines a central space **15** which lies inside it. Situated beneath the central space **15** is a reservoir chamber **31** which supplies liquid to an aerosol generator. The inner wall **12** therefore acts as a barrier which prevents liquid from flowing from the filling chamber to the reservoir chamber.

The aerosol generator may be, for example, a vibrating perforated membrane **34.** The membrane **34** has a large number of holes, typically from about 1 µm to about 10 µm in diameter at the exit (aerosol) side of the membrane. Without vibration of the membrane, the balance of pressures, the shape of the holes and the nature of the material used for the membrane are such that the liquid does not seep out through the membrane. However, vibration of the membrane leads to the formation and emission of aerosol droplets through the holes.

The dosing system has a plunger **20** for insertion into the filling chamber. The plunger has an outer wall **21,** an inner wall **22** and a base **23** which connects the outer and inner walls. Together, the walls and base form an overflow chamber **25.** In contrast to some of the dosing systems of WO 2015/022436, the plunger is not (and cannot be) inserted into the reservoir chamber. This is advantageous because there is no risk of the plunger being forced too far into the reservoir chamber and coming in to contact with the membrane **34.**

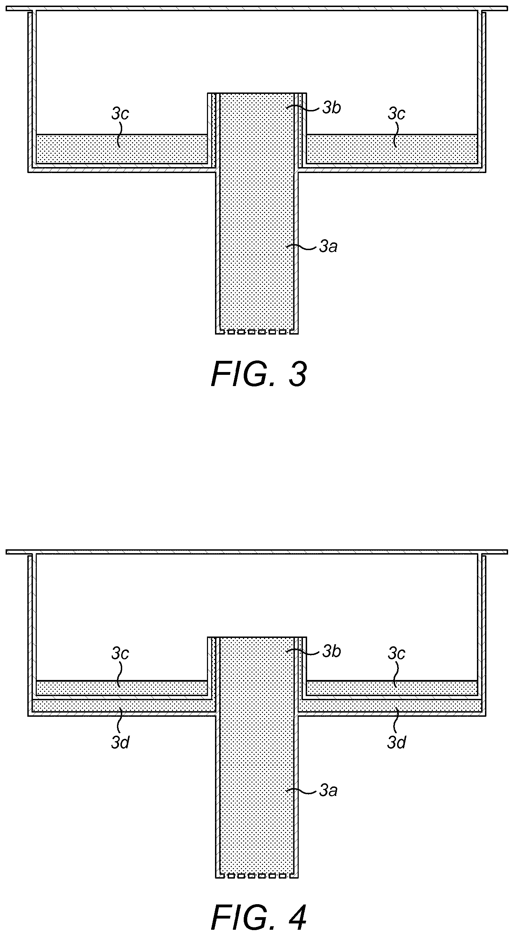

The plunger is also annular (when viewed from above) and corresponds to the size and shape of the filling chamber, so that they form a close fit when the plunger is inserted. Nonetheless, there is a small gap **26** between inner walls **12, 22** of the filling chamber and plunger through which the liquid displaced from the filling chamber flows. The gap may be from 0.1 to 0.2mm in size, such as about 0.15mm. The top of the inner wall **12** of the filling chamber and the top of the inner wall **22** of the plunger **20** are at the same height when the plunger is fully inserted, as shown in Figure 3.

The plunger has a lid **27** which covers the top of the overflow chamber **25.** The lid hides the excess liquid from the user after nebulization. In contrast, in the known dosing systems described above, the excess liquid is visible to the user once the plunger is been removed after nebulization. The presence of visible liquid may confuse the user, who may think that this liquid should have been nebulized, and who therefore may be tempted to try to pour the excess liquid back into the filling chamber, and hence dose more than the correct amount.

The dosing system operates as follows. The liquid **3** is poured into the filling chamber **10,** for example from a cartridge or ampoule (Figure 1). The inner wall **12** prevents liquid from flowing directly into the reservoir chamber **31.** When the plunger **20** is inserted (Figure 2), some of the liquid in the filling chamber **10** is displaced through the gap **26** between the inner walls of the filling chamber and plunger, over the filling chamber inner wall **12,** through the outlet opening **14** and into the reservoir chamber **31.** Once the reservoir chamber **31** and the central space **15** inside the inner wall **12** of the filling chamber is full of liquid (**3a, 3b** respectively), the remaining liquid in the filling chamber **10** is displaced over the inner wall **22** of the plunger **20** and into the overflow chamber **25.** Once the plunger has been fully inserted (Figure 3), if a small residual amount of liquid (e.g. the liquid from the gap **26**) remains in the filling chamber, it is prevented by the inner wall **12** from entering the reservoir chamber **31** and therefore being inadvertently nebulized. The liquid **3a** in the reservoir chamber **31** together with the liquid **3b** in the central space **15** is available to be nebulized.

The excess liquid **3c** is isolated and retained in the overflow chamber **25,** and cannot be nebulized. The dosing system thus dispenses a metered volume of liquid (**3a** + **3b**) to the aerosol generator.

After nebulization has been completed, the excess liquid is poured out of the overflow chamber. The dosing system can then be rinsed out before the next use. The plunger, filling chamber or the whole dosing system may be removable from the nebulizer device so that it can be cleaned by the user, for example rinsed with water and / or placed into a dishwasher.

In Figures 1 to 3, the plunger **20** occupies essentially the whole of the filling chamber **10** when fully inserted (apart from the gap **26**), so that little or no residual liquid remains in the filling chamber. In a variant shown in Figure 4, the plunger **20** occupies less than the whole volume of the filling chamber **10** when fully inserted so that a portion of the excess liquid **3c** is displaced into the overflow chamber, whilst another residual portion **3d** remains in the filling chamber. This residual liquid **3d** is prevented by the inner wall **12** from entering the reservoir chamber **31** during nebulization. However, this is less preferred, as after nebulization has been completed, the user may see the liquid **3d** remaining in the filling chamber and think that it should have been nebulized.

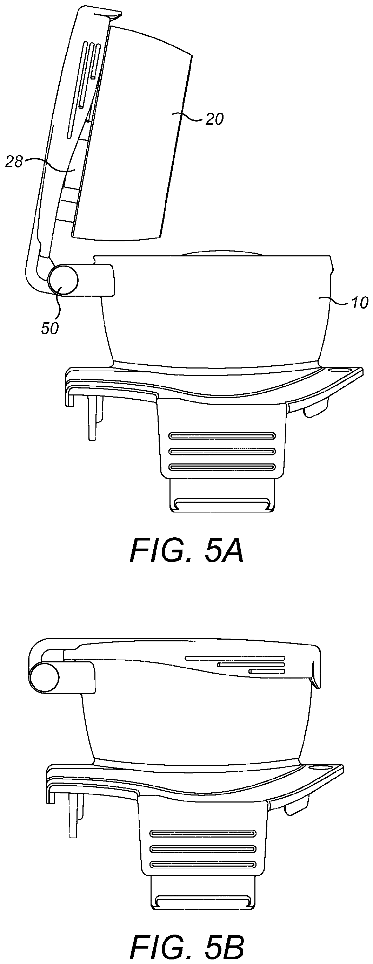

In the schematic views of Figures 1 to 4, the plunger is shown as being inserted linearly downwards into the filling chamber for simplicity. However, in the dosing system of the invention, shown in Figures 5 to 9, the plunger is inserted and removed by a pivoting motion about a hinge **50.** This results in the plunger being inserted at a slight angle. Consequently, parts of the walls of the plunger and filling chamber are shaped as appropriate matching curves. The connection between the plunger and the hinge may be designed to allow an easy exchange of the plunger, while at the same time preventing accidental loss of the plunger.

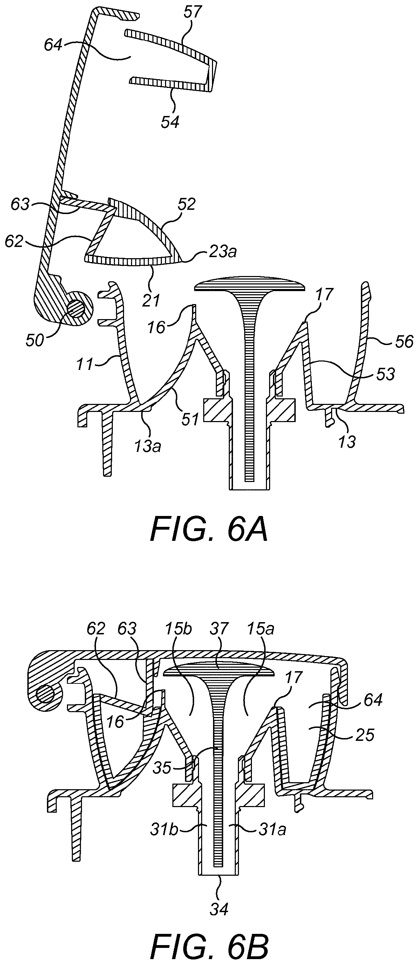

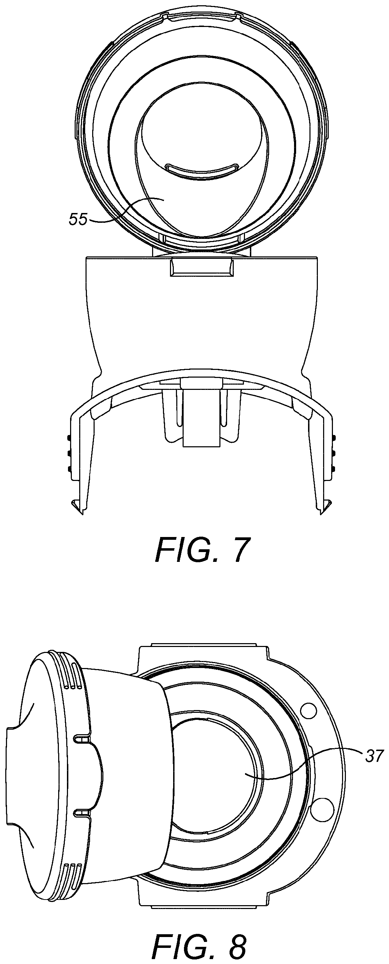

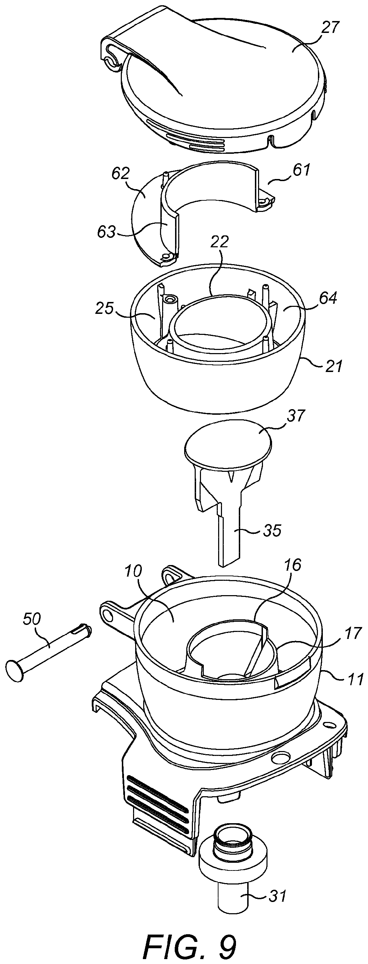

Figures 5A and 5B show side views before and after the plunger **20** is inserted into the filling chamber **10** respectively. Figures 6A and 6B show the corresponding cross-sectional views. Figures 7 and 8 are views of the dosing system in the open position (i.e. before insertion of the plunger) from the front and from above respectively. Figure 9 is an expanded view showing the components of the dosing system.

As can be seen in Figure 6B, the inner walls of the filling chamber and the plunger are curved **51, 52** in the region close to the hinge **50,** so that the plunger can be pivoted into the filling chamber whilst also ensuring a close fit between the inner walls when the plunger is fully inserted. Nonetheless, the plunger does not need to form a pressure-tight seal with the filling chamber, and indeed intentionally does not do so, in order to provide a gap through which the liquid displaced from the filling chamber can flow.

Moreover, since there is no need for a seal, unlike the dosing systems of WO 2015/022436, the plunger and / or filling chamber do not need to be made from flexible materials, nor are additional components, such as O rings, required. Thus the construction of the dosing system is simplified. Conveniently therefore, the plunger and filling chamber are made from a rigid material, preferably a rigid plastic material.

As a result of the curvature, the bottom of the curved part **51** of the inner wall of the filling chamber meets the outer wall **11** on the side closest to the hinge (shown in Figure 6A), so that it also effectively forms the base **13a** of the filling chamber in this region. Correspondingly, the bottom of the curved part **52** of the inner wall of the plunger also meets the outer wall **21** on the side closest to the hinge and forms the base of the plunger **23a** in this region. Thus the curved part of the inner wall of the plunger **52** forms a cut-away region **55** in the annular shape of the plunger, as shown in Figure 7. In contrast, the inner walls of the filling chamber and the plunger are not constrained by the pivoting motion on the side opposite the hinge **53, 54,** and therefore can be straight.

The outer wall **11** of the filling chamber and the outer wall **21** of the plunger are curved in their respective regions **56, 57** where they are furthest from the hinge **50** for the same reasons. Since they are further from the hinge, the radius of curvature is greater for the curved regions **56, 57** of the outer walls than for the curved regions **51, 52** of the inner walls. Although not necessary for the pivoting motion, the outer walls may have the same curvature everywhere for simplicity and aesthetic appearance. Thus, as shown in Figure 9, the outer walls **11, 21** of the filling chamber and plunger are both curved, with a small increase in diameter from the base to the top as a result of the shaping.

Inserting the plunger too rapidly could cause liquid to splash out of the filling chamber, rather than steadily displacing it into the reservoir chamber and overflow chamber. In order to prevent this, the hinge may be provided with a detent mechanism which resists the pivoting motion of the plunger. The detent mechanism may operate over the whole pivoting motion, or only in the latter part, i.e. as the plunger comes into contact with the liquid in the filling chamber. The detent mechanism may be any mechanism which is capable of applying a biasing force to the plunger, e.g. a spring or a cam follower associated with the plunger and a corresponding track associated with the filling chamber.

Due to the pivoting motion, the overflow chamber is rotated through approximately 90 degrees when the plunger is moved back into the open position after nebulization. In order to prevent the excess liquid from flowing out of the overflow chamber in this position, there is a cover **61** between the inner **22** and outer **21** walls on the side adjacent to the hinge, visible in Figures 6 and 9. The cover comprises a semi-annular floor **62** and a corresponding semi-annular wall **63.**

When the overflow chamber **25** is in the open position (Figure 6A), the cover floor **62** is in a generally vertical orientation. The cover floor must close enough of the overflow chamber so that the liquid cannot flow out when the plunger is pivoted into the open position. For example, as is apparent from Figure 9, the cover **61** suitably closes the top of the overflow chamber **25** on the side adjacent to the hinge **50** to prevent liquid from flowing out of the overflow chamber, whereas the other side **64** is uncovered.

The filling chamber inner wall is higher **16** on the side adjacent to the hinge than on the opposite side **17** (see Figures 6 and 9). Thus when the plunger is inserted, excess liquid is displaced over the lower side **17** of the filling chamber inner wall and enters the open (uncovered) region **64** of the overflow chamber on the side opposite the hinge. Liquid may also be displaced upwards between the filling chamber outer wall and the overflow chamber outer wall. In order to ensure that this liquid does not seep out of the dosing system, the filling chamber outer wall **11** is higher than the overflow chamber outer wall **21,** as shown in Figure 6B.

The higher part **16** of the filling chamber inner wall, together with the cover wall **63** prevents liquid from flowing onto the top of the cover floor **62,** or at least minimizes the amount of liquid that does so. Nonetheless, the cover floor **62** is not exactly horizontal (in the closed position) but instead slopes downwardly away from the hinge **50.** Consequently any liquid which passes over or round the higher part of the filling chamber inner wall, or over the filling chamber outer wall, and onto the top of the cover floor **62,** is guided back down the slope towards the open (uncovered) region **64** and into the overflow chamber **25.**

The plunger **20** has a fixed lid **27** which covers the top of the overflow chamber **25,** so that the user cannot put the liquid directly into the overflow chamber by mistake. The plunger has an overflow chamber outlet opening **28,** shown in Figure 5, which extends around the whole circumference of the outer wall without a spout. The outlet opening **28** makes it possible, but somewhat awkward, for the user to pour the excess liquid out of the overflow chamber after nebulization has been completed, in order to emphasize that the excess liquid is not intended to be re-used.

Alternatively, the lid may be separable from the overflow chamber in order to facilitate emptying and cleaning of the overflow chamber after use. For example, the lid and overflow chamber may be separately pivotable. The overflow chamber and lid may have clip formations so that when the lid is closed, it becomes attached to the overflow chamber. Thus, after nebulization has been completed, the lid and overflow chamber are pivoted together, and the excess liquid in the overflow chamber is not visible to the user. When the plunger is in the open position, the clip formations can be detached from each other, so that the user can then open the lid, in order to pour out the excess liquid and clean the overflow chamber. The action of having to unclip the lid before pouring out the excess liquid acts as a reminder to the user that the excess liquid is not intended to be re-used.

The whole dosing system can be removed from the nebulizer so that the filling chamber, overflow chamber and reservoir chamber can be cleaned by the user, for example rinsed with water and / or placed into a dishwasher.

As shown in Figures 6 and 9, the central space **15** inside the inner wall of the filling chamber is divided by a partition **35** which is parallel to the hinge and extends vertically down into the reservoir chamber **31,** thereby separating the central space **15** and reservoir chamber **31** into two parts (**15a, 31a** and **15b, 31b** respectively). The partition however does not extend all the way to the membrane **34,** so that the reservoir chamber is not divided at the bottom. Since the inner wall of the filling chamber is higher **16** on one side of the partition, and lower **17** on the other, the liquid which is displaced from the filling chamber **10** when the plunger **20** is inserted flows over the lower part **17** of the inner wall and down the corresponding side of the central space **15a** and reservoir chamber **31a** to the bottom of the reservoir chamber **31.** At the same time, air is displaced upwards on the other side of the reservoir chamber **31b** and the central space **15b,** and over the higher part **16** of the inner wall. This prevents the formation of an airlock, i.e. a trapped bubble of air at the bottom of the reservoir chamber.

The partition **35** occupies part of the volume defined by the reservoir chamber **31** and the central space **15,** and thus reduces the free volume which can be occupied by the liquid. The volume of liquid dispensed is given by the volume of the reservoir chamber plus the volume of the central space up to the lower part of the filling chamber wall minus the volume of these which is occupied by the partition. The partition therefore provides a further advantage, namely the ability to dispense smaller volumes of liquid than would otherwise be possible. The thickness and or length of the partition can be chosen according to the desired volume of liquid to be nebulized.

The central space **15** inside the filling chamber inner wall **12** may be covered with a cap **37** (see Figures 6, 8 and 9). This prevents the user from dosing liquid directly into the reservoir chamber **31** whilst allowing liquid to be dosed into the filling chamber. Consequently (and unlike those dosing systems of WO 2015/022436 in which there is a fixed barrier between the filling chamber and the reservoir chamber), there is no need for a mechanism (such as a safety plunger) to prevent accidental overfilling of the reservoir chamber. Conveniently, the cap **37** can be formed as an extension of the partition **35.**

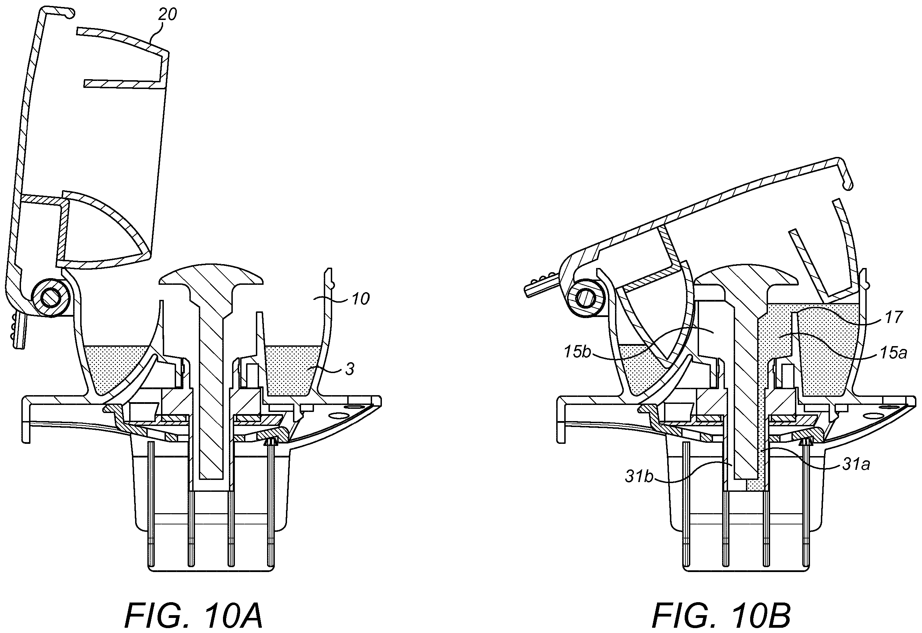

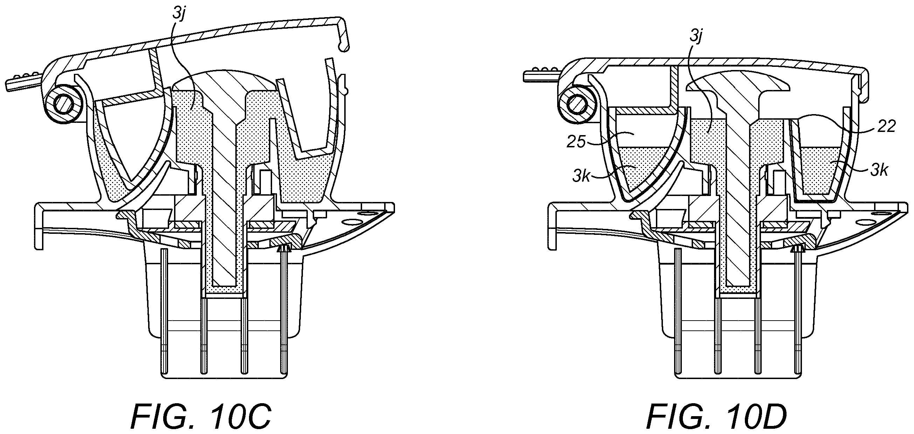

Figure 10 shows the dosing system of Figures 5 to 9 in operation. In Figure 10A, the plunger **20** is in the open position and the filling chamber **10** contains liquid **3.** In Figure 10B, the plunger **20** has been partly closed and partially inserted into the filling chamber **10,** so that it has displaced some of the liquid **3** over the lower side of the inner wall **17** and into the central space **15a,** from where it flows down into the reservoir chamber **31a.** Air is displaced up the other side **31b, 15b.** As the plunger is pivoted further into the filling chamber, the central space and reservoir chamber are filled with liquid **3j,** shown in Figure 10C. Thereafter, the remaining (i.e. excess) liquid **3k** is displaced from the filling chamber over the plunger inner wall **22** and into the overflow chamber **25** until the plunger has been completely inserted, shown in Figure 10D.

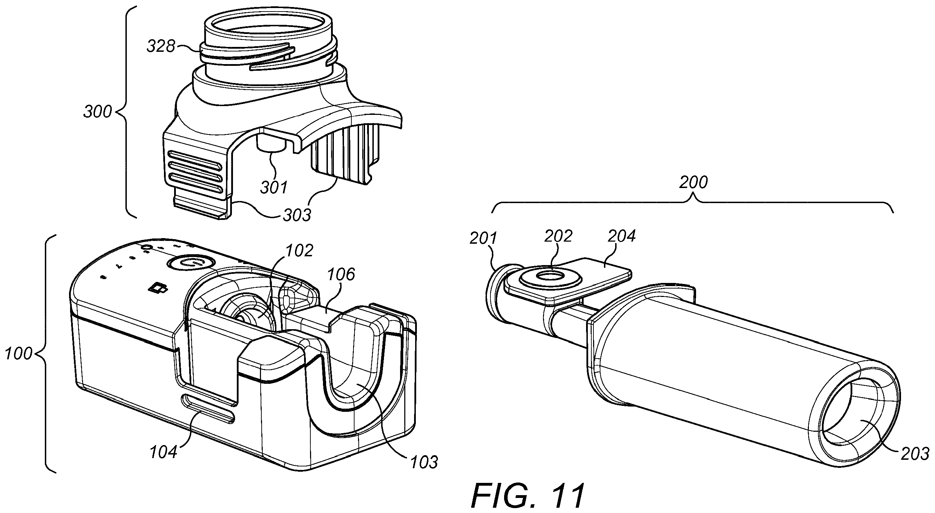

The dosing system is suitable for use with the nebulizer device shown in Figure 11, which is described in detail in EP 2 724 741. The device comprises three parts: a base unit, a mouthpiece, and an aerosol head. The base unit **100** has one or more air inlet opening(s), an air outlet opening **102,** a groove **103** for receiving the mouthpiece **200,** and one or more key lock members **104.** The mouthpiece **200** has an air inlet opening **201** which is attachable to the air outlet opening **102** of the base unit **100,** a lateral opening **202** for receiving an aerosol generator **301,** and an aerosol outlet opening **203.** The mouthpiece **200** is insertable into the groove **103** of the base unit **100.** The aerosol head **300** has an aerosol generator **301** and one or more key lock members **303** complementary to the key lock members **104** of the base unit **100.** In Figure 11, the dosing system is not shown, but may be attached to the aerosol head **300** by means of a screw thread **328.**

The base unit **100,** the mouthpiece **200** and the aerosol head **300** are detachably connectible with one another. The device is assembled by inserting the mouthpiece **200** into the groove **103** in the base unit **100,** then placing the aerosol head **300** over the mouthpiece **200** and engaging the key lock member(s) **303** of the aerosol head **300** with the complementary member(s) **104** of the base unit **100** by gentle pressure on both the aerosol head and the base unit. The aerosol generator **301** is positioned in the aerosol head **300** in such a way that when engaging the key lock member(s), the aerosol generator **301** is inserted into the lateral opening **202** of the mouthpiece **200.** This creates airtight connections between the aerosol generator **301** and the lateral opening **202** in the mouthpiece as well as between the air outlet opening **102** of the base unit **100** and the air inlet opening **201** of the mouthpiece **200.** The base unit **100,** the mouthpiece **200** and the aerosol head **300** can be separated by reversing these steps.

The base unit **100** may have one or more indentation(s) **106** whose position may be at or near the groove **103,** and the mouthpiece **200** may have one or more positioning member(s) **204.** The indentation(s) of the base unit are complementary to (i.e. shaped to receive) the positioning member (s) **204** of the mouthpiece **200.** In this context, an indentation is a depression (e.g. a recess, pit, cavity, void, notch or the like) whose "negative" shape is complementary to the "positive" shape of a positioning member (which may be a flange, projection, nose, bulge or the like). Together, such indentations and positioning members act to position the mouthpiece correctly in the base unit. The indentation(s) **106** and the positioning member(s) **204** may be asymmetrical, so as to ensure that the mouthpiece **200** can only be inserted into the indentation **106** of the base unit **100** in one particular manner. This ensures that the device is assembled in such a way that the position and orientation of the mouthpiece **200** and base unit **100** relative to each other are correct.

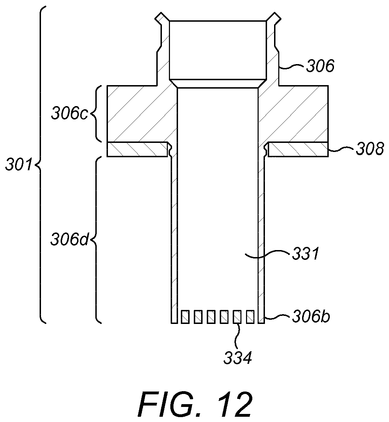

The aerosol generator **301** is preferably an ultrasonic liquid atomiser comprising a piezoelectric member **308** and a transducer body **306** as shown in Figure 12 and described in WO 2008/058941. The transducer body **306** is made of e.g. stainless steel, titanium or aluminium, and encloses the reservoir chamber **331.** The reservoir chamber **331** is connected to the dosing system (not shown in Figure 12) so as to receive liquid to be nebulized from it.

The piezoelectric member **308** is preferably an annular single or multilayer ceramic, which vibrates the transducer body **306** in a longitudinal mode, at a frequency preferably in the 50 to 200 kHz range. As a result, micronic longitudinal displacements, or deformations, occur in a direction parallel to the symmetry axis of the transducer body **306.** The transducer body **306** has a region close to the piezoelectric member **308** with a relatively large wall thickness, which serves as a stress concentration zone **306c,** and a region downstream thereof **306d** with a relatively low wall thickness which serves as a deformation amplification zone. In this configuration, the vibrations or deformations of the transducer body **306** caused by the piezoelectric member **308** are amplified. Preferably, the piezoelectric member **308** is located at the level of, or adjacent to, the stress concentration zone **306c.** The internal diameter of the transducer body **306** at the deformation amplification zone **306d** may be the same as at the stress concentration zone **306c,** so that the differences in wall thickness correspond to different external diameters. Alternatively, the external diameter of the transducer body **306** may be constant, while the inner diameters differ at the position of the two zones.

A perforated membrane **334** is positioned at the downstream end **306b** of the transducer body **306.** The holes may be formed by electroforming or by laser drilling, with openings normally being in the range from about 1 µm to about 10 µm. Without vibration of the membrane, the balance of pressures, the shape of the holes and the nature of the material used for the membrane are such that the liquid does not seep out through the membrane. However, vibration of the membrane leads to the formation and emission of aerosol droplets through the holes. The membrane may be made of plastic, silicon, ceramic or more preferably metal, and may be affixed to the downstream end **306b** of the aerosol generator **301** by various means, such as gluing, brazing, crimping or laser welding. Optionally, the membrane at least partially forms a dome in its central region, which causes the jet of nascent aerosol droplets to diverge and hence reduces the risk of droplet coalescence.

Once a treatment operation has been completed, the aerosol head key lock members **303** are disengaged from the complementary member(s) **104** of the base unit, so that the aerosol generator **301** can be removed from the lateral opening **202** of the mouthpiece.

A patient may receive two (or more) different drugs, which will generally require different volumes of liquid to be dispensed, and different aerosolisation parameters, such as droplet size, treatment time etc.. Thus a patient may have two (or more) different nebulization devices which are adapted for the different drugs. The first aerosol head has a dosing system designed to dispense the appropriate volume of liquid and the first base unit is configured to provide the appropriate aerosolisation parameters for the first drug. Similarly the second aerosol head and base unit are configured to dispense and aerosolize the second drug. A recognition system can be provided to ensure that the patient uses the correct combinations of aerosol head and base unit. The recognition system could be, for example, based on RFID tags, electrical contacts or mechanical interlock.

A simple mechanical recognition system consists of complementary male and female features on the aerosol head and base unit, for example, one or more cavities / holes on the aerosol head and corresponding protrusions / pegs on the base unit. These may be present in one or more locations and / or sizes and / or shapes selected from a pre-determined number of locations and / or sizes and / or shapes. Conveniently, the complementary features can be located on or formed as part of the key lock members **104, 303.** Alternatively the complementary features may be on other parts of the aerosol head and base unit.

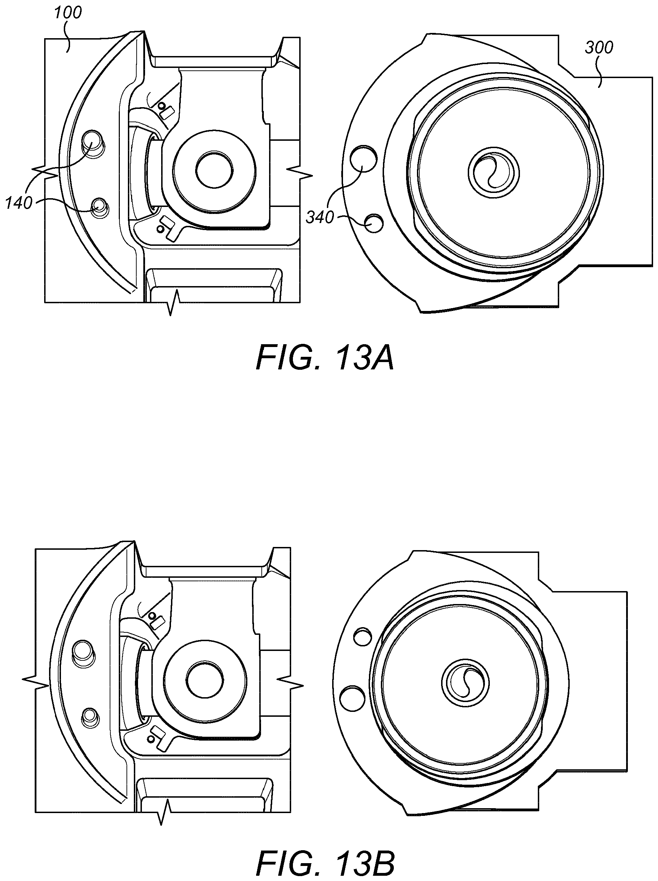

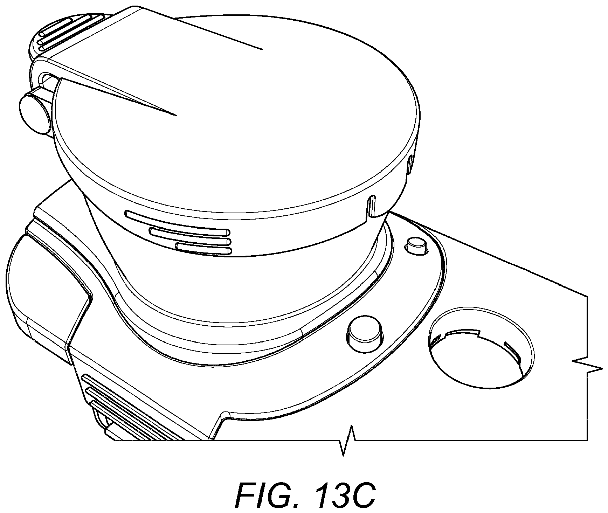

Figure 13 shows an example of a recognition system which has several (e.g. five) potential hole locations on the aerosol head **300** and corresponding potential peg locations on the base unit **100.** Each hole and peg can be either large or small, in order to maximise the number of possible variants for a given number of potential locations. In each variant, two holes **340** are present, one large and one small. The base unit **100** has two pegs **140,** also one large and one small. If the locations and sizes of the holes and pegs match (Figure 13A) then the aerosol head interlocks with, and fits onto, the base unit (Figure 13C). An advantage of this system is that the pegs and holes are visible, so that the user can easily judge whether the aerosol head and base unit will fit together, i.e. are complementary. Nevertheless, if the user does attempt to use an incorrect aerosol head for the base unit, then the pegs do not match the holes (Figure 13B). In this event, the pegs **140** hold the aerosol head **300** slightly apart from the base unit **100** which prevents the respective key lock members from interlocking with each other. Thus the recognition system provides both a strong visual cue for the correct combination of the aerosol head and base unit, and also a failsafe mechanism which prevents incorrect combinations from being formed.

## Claims

1. A dosing system (1) for an inhalation device, the dosing system comprising:
(a) a filling chamber (10) for receiving a liquid (3) to be aerosolized, the filling chamber having an outer wall (11), a base (13) and an inner wall (12) which defines an outlet opening (14);
(b) a reservoir chamber (31) for supplying liquid to an aerosol generator (34); and
(c) a plunger (20) which is mounted on a hinge (50) and which includes an overflow chamber (25),
wherein the inner wall (12) of the filling chamber (10) is higher on the side (16) adjacent to the hinge (50) than on the opposite side so that when the filling chamber is filled with liquid (3) and the plunger (20) is inserted into the filling chamber by pivoting it about the hinge (50), part of the liquid is displaced by the plunger over the lower side (17) of the inner wall of the filling chamber and into the reservoir chamber (31) via the outlet opening (14), and some or all of the remaining liquid is displaced by the plunger from the filling chamber into the overflow chamber (25).

2. A dosing system according to claim 1 comprising a cap (37) located above the filling chamber outlet opening (14) which prevents liquid from being supplied directly into the reservoir chamber (31).

3. A dosing system according to claim 1 or 2, wherein the plunger (20) has an inner wall (22) and an outer wall (21), and wherein at least part of the inner and outer walls of the filling chamber and the plunger are curved so that there is a close fit between the inner walls of the plunger and filling chamber when the plunger is fully inserted into the filling chamber.

4. A dosing system according to any of claims 1 to 3 comprising a partition (35) located inside the inner wall (12) of the filling chamber which extends down into the reservoir chamber (31).

5. A dosing system according to claim 4 and claim 2 wherein the cap (37) is formed as an extension of the partition (35).

6. A dosing system according to any of claims 1 to 5 wherein the overflow chamber (25) has a cover (61) which closes the top of the overflow chamber on the side adjacent to the hinge (50).

7. A dosing system according to any of claims 1 to 6, wherein the plunger (20) has a lid (27).

8. A dosing system according to claim 7, wherein the lid (27) is fixed and there is an opening (28) between the lid and the top of the overflow chamber.

9. A dosing system according to claim 7, wherein the overflow chamber (25) and the lid (27) are separately pivotable, and preferably wherein the overflow chamber and lid have clip formations so that when the lid is closed, it becomes attached to the overflow chamber.

10. A dosing system according to any of claims 1 to 9, wherein the overflow chamber (25) corresponds to the size and shape of the filling chamber (10), so that the plunger (20) occupies substantially the whole of the filling chamber when inserted.

11. A dosing system according to any of claims 1 to 10 wherein the filling chamber (10) and the plunger (20) are made from a rigid material, such as a rigid plastic.

12. An inhalation device comprising a dosing system (1) according to any of claims 1 to 11.

13. An inhalation device according to claim 12 comprising: an aerosol head (300) comprising the dosing system (1); and a base unit (100), wherein the aerosol head and base unit are detachably connectible with each other and wherein the aerosol head and base unit have complementary male and female features (140, 340) which interlock to provide a recognition system.

14. An inhalation device according to claim 13 wherein the base unit (100) has two pegs (140) of different sizes and the aerosol head (300) has two corresponding holes (340).

15. A method for dosing liquid to an inhalation device according to any of claims 12 to 14, the method comprising:
supplying a liquid (3) to be aerosolized to the filling chamber (10); and
inserting the plunger (20) into the filling chamber so that part of the liquid is displaced over the lower side (17) of the inner wall of the filling chamber into the reservoir chamber (31) and some or all of the remaining liquid is displaced into the overflow chamber (25).

## Patentansprüche

1. Dosiersystem (1) für eine Inhalationsvorrichtung, wobei das Dosiersystem umfasst:
(a) eine Füllkammer (10) zum Aufnehmen einer zu aerosolisierenden Flüssigkeit (3), wobei die Füllkammer eine Außenwand (11), eine Basis (13) und eine Innenwand (12) aufweist, die eine Auslassöffnung (14) definiert;
(b) eine Vorratskammer (31) zum Zuführen von Flüssigkeit zu einem Aerosolerzeuger (34); und
(c) einen Kolben (20), der auf einem Scharnier (50) montiert ist und eine Überlaufkammer (25) enthätt,
wobei die Innenwand (12) der Füllkammer (10) auf der an das Scharnier (50) angrenzenden Seite (16) höher als auf der gegenüberliegenden Seite ist, sodass, wenn die Füllkammer mit Flüssigkeit (3) gefüllt ist und der Kolben (20) durch Schwenken um das Scharnier (50) in die Füllkammer eingeführt wird, ein Teil der Flüssigkeit durch den Kolben über die Unterseite (17) der Innenwand der Füllkammer und über die Auslassöffnung (14) in die Vorratskammer (31) verdrängt wird und ein Teil oder die gesamte restliche Flüssigkeit durch den Kolben aus der Füllkammer in die Überlaufkammer (25) verdrängt wird.

2. Dosiersystem gemäß Anspruch 1, umfassend eine Kappe (37), die sich oberhalb der Füllkammerauslassöffnung (14) befindet und verhindert, dass Flüssigkeit direkt in die Vorratskammer (31) zugeführt wird.

3. Dosiersystem gemäß Anspruch 1 oder 2, wobei der Kolben (20) eine Innenwand (22) und eine Außenwand (21) aufweist und wobei mindestens ein Teil der Innen- und Außenwand der Füllkammer und des Kolbens so gekrümmt sind, dass eine enge Passung zwischen den Innenwänden des Kolbens und der Füllkammer besteht, wenn der Kolben vollständig in die Füllkammer eingeführt ist.

4. Dosiersystem gemäß einem der Ansprüche 1 bis 3, umfassend eine Trennwand (35), die innerhalb der Innenwand (12) der Füllkammer angeordnet ist und sich nach unten in die Vorratskammer (31) erstreckt.

5. Dosiersystem gemäß Anspruch 4 und Anspruch 2, wobei die Kappe (37) als eine Verlängerung der Trennwand (35) ausgebildet ist.

6. Dosiersystem gemäß einem der Ansprüche 1 bis 5, wobei die Überlaufkammer (25) eine Abdeckung (61) aufweist, der die Oberseite der Überlaufkammer auf der an das Scharnier (50) angrenzenden Seite verschließt.

7. Dosiersystem gemäß einem der Ansprüche 1 bis 6, wobei der Kolben (20) einen Deckel (27) aufweist.

8. Dosiersystem gemäß Anspruch 7, wobei der Deckel (27) feststehend ist und eine Öffnung (28) zwischen dem Deckel und der Oberseite der Überlaufkammer vorhanden ist.

9. Dosiersystem gemäß Anspruch 7, wobei die Überlaufkammer (25) und der Deckel (27) separat schwenkbar sind und wobei die Überlaufkammer und der Deckel vorzugsweise Klammerausbildungen aufweisen, sodass, wenn der Deckel geschlossen wird, dieser an der Überlaufkammer befestigt wird.

10. Dosiersystem gemäß einem der Ansprüche 1 bis 9, wobei die Überlaufkammer (25) der Größe und Form der Füllkammer (10) entspricht, sodass der Kolben (20) beim Einführen im Wesentlichen die gesamte Füllkammer einnimmt.

11. Dosiersystem gemäß einem der Ansprüche 1 bis 10, wobei die Füllkammer (10) und der Kolben (20) aus einem starren Material, wie z. B. einem starren Kunststoff, hergestellt sind.

12. Inhalationsvorrichtung, umfassend ein Dosiersystem (1) gemäß einem der Ansprüche 1 bis 11.

13. Inhalationsvorrichtung gemäß Anspruch 12, umfassend:
einen Aerosolkopf (300), der das Dosiersystem (1) umfasst;
und eine Basiseinheit (100), wobei der Aerosolkopf und die Basiseinheit lösbar miteinander verbindbar sind und wobei der Aerosolkopf und die Basiseinheit einander ergänzende Steck- und Buchsenmerkmale (140, 340) aufweisen, die ineinandergreifen, um ein Erkennungssystem bereitzustellen.

14. Inhalationsvorrichtung gemäß Anspruch 13, wobei die Basiseinheit (100) zwei Stifte (140) mit verschiedenen Größen aufweist und der Aerosolkopf (300) zwei entsprechende Löcher (340) aufweist.

15. Verfahren zum Dosieren von Flüssigkeit zu einer Inhalationsvorrichtung gemäß einem der Ansprüche 12 bis 14, wobei das Verfahren umfasst:
Zuführen einer zu aerosolisierenden Flüssigkeit (3) in die Füllkammer (10); und
Einführen des Kolbens (20) in die Füllkammer, sodass ein Teil der Flüssigkeit über die Unterseite (17) der Innenwand der Füllkammer in die Vorratskammer (31) verdrängt wird und ein Teil oder die gesamte restliche Flüssigkeit in die Überlaufkammer (25) verdrängt wird.

## Revendications

1. Système de dosage (1) pour un dispositif d'inhalation, le système de dosage comprenant :
(a) une chambre de remplissage (10) pour recevoir un liquide (3) destiné à être transformé en aérosol, la chambre de remplissage ayant une paroi extérieure (11), une base (13) et une paroi intérieure (12) qui définit une ouverture de sortie (14) ;
(b) une chambre de réservoir (31) pour alimenter, en liquide, un générateur d'aérosol (34) ; et
(c) un piston plongeur (20) qui est monté sur une articulation (50) et qui inclut une chambre de trop-plein (25),
dans lequel la paroi intérieure (12) de la chambre de remplissage (10) est plus élevée sur le côté (16) adjacent à l'articulation (50) que sur le côté opposé pour que, lorsque la chambre de remplissage est remplie avec le liquide (3) et le piston plongeur (20) est inséré dans la chambre de remplissage en le faisant pivoter autour de l'articulation (50), une partie du liquide soit déplacée par le piston plongeur par-dessus le côté inférieur (17) de la paroi intérieure de la chambre de remplissage et dans la chambre de réservoir (31) par l'intermédiaire de l'ouverture de sortie (14), et une certaine quantité ou la totalité du liquide restant soit déplacée par le piston plongeur à partir de la chambre de remplissage dans la chambre de trop-plein (25).

2. Système de dosage selon la revendication 1, comprenant un capuchon (37) situé au-dessus de la ouverture de sortie de chambre de remplissage (14) qui empêche l'alimentation directe en liquide dans la chambre de réservoir (31).

3. Système de dosage selon la revendication 1 ou 2, dans lequel le piston plongeur (20) a une paroi intérieure (22) et une paroi extérieure (21), et dans lequel au moins une partie des parois intérieure et extérieure de la chambre de remplissage et le piston plongeur sont incurvés pour qu'il y ait un ajustement serré entre les parois intérieures du piston plongeur et la chambre de remplissage lorsque le piston plongeur est complètement inséré dans la chambre de remplissage.

4. Système de dosage selon l'une quelconque des revendications 1 à 3, comprenant une cloison (35) située à l'intérieur de la paroi intérieure (12) de la chambre de remplissage qui s'étend vers le bas dans la chambre de réservoir (31).

5. Système de dosage selon la revendication 4 et la revendication 2, dans lequel le capuchon (37) est sous forme d'extension de la cloison (35).

6. Système de dosage selon l'une quelconque des revendications 1 à 5, dans lequel la chambre de trop-plein (25) a un couvercle (61) qui ferme le haut de la chambre de trop-plein sur le côté adjacent à l'articulation (50).

7. Système de dosage selon l'une quelconque des revendications 1 à 6, dans lequel le piston plongeur (20) a une coiffe (27).

8. Système de dosage selon la revendication 7, dans lequel la coiffe (27) est fixe et il y a une ouverture (28) entre la coiffe et le haut de la chambre de trop-plein.

9. Système de dosage selon la revendication 7, dans lequel la chambre de trop-plein (25) et la coiffe (27) sont pivotables séparément, et de préférence dans lequel la chambre de trop-plein et la coiffe ont des formations d'attache pour que, lorsque la coiffe est fermée, elle soit attachée à la chambre de trop-plein.

10. Système de dosage selon l'une quelconque des revendications 1 à 9, dans lequel la chambre de trop-plein (25) correspond à la taille et la forme de la chambre de remplissage (10), pour que le piston plongeur (20) occupe sensiblement l'ensemble de la chambre de remplissage lorsque inséré.

11. Système de dosage selon l'une quelconque des revendications 1 à 10, dans lequel la chambre de remplissage (10) et le piston plongeur (20) sont faits d'un matériau rigide, tel qu'un plastique rigide.

12. Dispositif d'inhalation, comprenant un système de dosage (1) selon l'une quelconque des revendications 1 à 11.

13. Dispositif d'inhalation selon la revendication 12, comprenant :
une tête d'aérosol (300) comprenant le système de dosage (1) ; et
une unité de base (100), dans lequel la tête d'aérosol et l'unité de base sont peuvent être raccordées de façon détachable l'une à l'autre et dans lequel la tête d'aérosol et l'unité de base ont des dispositifs mâle et femelle complémentaires (140, 340) qui s'enclenchent pour fournir un système de reconnaissance.

14. Dispositif d'inhalation selon la revendication 13, dans lequel l'unité de base (100) a deux chevilles (140) de différentes tailles et la tête d'aérosol (300) a deux trous correspondant (340).

15. Procédé pour doser un liquide pour un dispositif d'inhalation selon l'une quelconque des revendications 12 à 14, le procédé comprenant :
l'alimentation en un liquide (3) destiné à être transformé en aérosol à la chambre de remplissage (10) ; et
l'insertion du piston plongeur (20) dans la chambre de remplissage pour qu'une partie du liquide soit déplacée par-dessus le côté inférieur (17) de la paroi intérieure de la chambre de remplissage dans la chambre de réservoir (31) et une certaine quantité ou la totalité du liquide restant soit déplacée dans la chambre de trop-plein (25) .
